# EUROPEAN PATENT APPLICATION

(11) **EP 2 452 575 A1**
(43) Date of publication of application: **16.05.2012**
(21) Application number: 10191207.9
(22) Date of filing: 15.11.2010
(51) Int. Cl.: A23L 1/29, A23L 1/30, A23L 1/305

(54) **Array of age-tailored nutritional formula with probiotics**

(71) Applicant: Nestec S.A., 1800 Vevey (CH)
(72) Inventor: Klassen, Petra Gerda, 1806, St. Legier (CH); Magliola, Corinne, 1009, Pully (CH)
(74) Representative: Corticchiato, Olivier

(57) **Abstract**

The present invention relates to nutritional compositions which are specifically designed to address the needs of infants and young children of at least 2 years of age. In particular, the invention provides a set of nutritional compositions for infants and young children, each nutritional composition having varying probiotic content. The set of the invention is specifically aimed at providing an optimal amount of probiotics to infants and young children over time and at each specific age.

## Description

### Field of the invention

The present invention relates to nutritional formulae. The nutritional formulae are specifically designed to address the needs of infants and young children of at least 0 to 2 years of age. The invention provides a set of nutritional compositions for infants and young children, each nutritional composition having a varying probiotic content. The set of nutritional compositions is specifically aimed at providing an optimal adapted load of probiotics to infants and young children over time and at each specific age.

### Background of the invention

Infant formulae, follow-up formulae and grown-up milks which are aimed at different age groups of 0 to 6 months, 6 months to 1 year and 1 year to 2 years respectively are known. These infant formulae, follow-up formulae and grown-up milks aim to meet the requirements of infants and young children at the different ages.

An age-tailored nutrition system for infants is described in international patent application publication No. WO 2009/068549, wherein a protein nature and content are adapted to specific age groups.

Recently, probiotics have been included in infant formulae to provide specific benefits such as gut maturation, protection, recovery from gut discomfort etc. However, the actual need of an infant/young child varies over time and cannot be matched by a unique probiotic load.

### Object of the invention

The present invention aims to provide an adapted nutrition system which takes into account the changing needs of infants and young children in at least in the first two years of life of the infants and young children.

There is a need for providing infants and young children with nutritional solutions that reflects the evolution of the needs of the infants and young children over time and in particular mimic the evolving nutritional quality and composition of breast milk over time.

Furthermore there is a need for compositions particularly adapted in terms of probiotic content, each composition or each composition's subset being adapted to address the needs of infants and young children of the given age, and more particularly in terms of probiotic content, probiotic load (quantity) and/or probiotic nature.

This object is solved by means of the independent claims. The dependent claims further develop the central idea of the invention.

### Summary of the invention

In a first aspect a set of nutritional compositions for infants and young children is disclosed. The set of nutritional compositions for infants and young children comprises a first composition for infants between 0 up to 6 months, a second composition for infants above 6 months up to 1 year, a third composition for young children above 1 year up to 2 years. The first composition and the second composition comprise probiotics. The first and second compositions differ from each other in an amount and/or nature of probiotics present.

In a further aspect a use of the set of nutritional compositions for infants and young children is disclosed. The set of nutritional compositions for infants and young children provides a health benefit to infants and young children. The health benefit is treatment or prevention of diarrhea, treatment or prevention of gut discomfort, weaning facilitation, maturation of the immune system, prevention or management of allergy, reducing cardiovascular diseases later in life, reducing risk of obesity, reducing risk of infections, ensuring a normal growth curve, improving or insuring optimal cognition, improved immune function and immune defenses, prevention of upper respiratory tract infections like *otitis media* or common cold.

In a further aspect a nutrition kit for infants and young children is disclosed. The nutrition kit for infants and young children comprises the set of nutritional compositions, wherein the nutritional compositions are packed in single dose units, such that each single dose unit comprises sufficient nutritional composition to prepare a single serving upon reconstitution with water.

In a further aspect a method for the manufacture of a set of nutritional compositions for infants and young children is disclosed. The method comprises the steps of preparing at least a first, a second and a third nutritional composition for infants between 0 up to 6 months, for infants above 6 months up to 1 year and for young children above 1 year up to 2 years. Selecting for at least the first and the second nutritional composition at least one probiotic according to age and/or according to a desired effect, then incorporating said selected probiotic to the corresponding nutritional composition, such that at least the first composition differs from at least the second composition in an amount and/or nature of probiotics present therein.

Finally, an infant and young child nutrition regimen is disclosed. The infant and young child nutrition regimen comprises feeding said infant between 0 up to 6 months a nutritional composition comprising probiotics such that the daily caloric intake is at least 400 kcal, preferably at least 435 kcal, feeding said infant above 6 months up to 1 year a nutritional composition comprising probiotics such that the daily caloric intake is at least 550 kcal, preferably at least 580 kcal; and feeding said young child above 1 year up to 2 years a nutritional composition comprising probiotics such that the daily caloric intake is at least 750 kcal, preferably at least 765 kcal. At least one of the compositions differs from at least another one of the compositions in a nature and/or amount of probiotics present therein.

### Detailed description

For a complete understanding of the present invention and the advantages thereof, reference is made to the following detailed description of the invention.

It should be appreciated that various embodiments of the present invention can be combined with other embodiments of the invention and are merely illustrative of the specific ways to make and use the invention and do not limit the scope of the invention when taken into consideration with the claims and the following detailed description.

In the present description, the following words are given a definition that should be taken into account when reading and interpreting the description, examples and claims.

Infant: according to the Commission Directive 2006/141/EC of 22 December 2006 on infant formulae and follow-on formulae, article 1.2 (a), the term "infants" means children under the age of 12 months.

Young Children: according to the Commission Directives 2006/141/EC of 22 December 2006 and/or 91/321/EEC of 14 May 1991 on infant formulae and follow-on formulae, article 1.2 (b), the term "young children" means children aged between one and three years.

Infant formulae: according to the Commission Directives 2006/141/EC of 22 December 2006 and/or 91/321/EEC of 14 May 1991 on infant formulae and follow-on formulae, article 1.2 (c), the term "infant formula" means foodstuffs intended for particular nutritional use by infants during the first four to six months of life and satisfying by themselves the nutritional requirements of this category of persons. It has to be understood that infants can be fed solely with infant formulas, or that the infant formula can be used by the carer as a complement of human milk. It is synonymous to the widely used expression "starter formula".

Follow-on formulae: according to the Commission Directives 2006/141/EC of 22 December 2006 and/or 91/321/EEC of 14 May 1991 on infant formulae and follow-on formulae, article 1.2 (d), the term "follow-on formulae" means foodstuffs intended for particular nutritional use by infants aged over four months and constituting the principal liquid element in a progressively diversified diet of this category of persons.

Probiotic: according to the paper Probiotics in Man and Animals, J. Appl Bacteriol. 66: 365-378, a probiotic is defined as a live microbial feed supplement which beneficially affects the host animal by improving its intestinal microbial balance.

The present invention relates to a set of nutritional compositions for infants and young children. The set of nutritional compositions is formed of at least three nutritional compositions aimed at least to three different age groups.

In the present invention, the nutritional compositions can be in a form of a powder to be reconstituted with water. The nutritional compositions can be in a form of a concentrate to be diluted. Upon reconstitution or dilution of the nutritional compositions an end product is preferably a liquid.

The set of nutritional compositions comprises a first composition for infants between 0 up to 6 months, a second composition for infants above 6 months up to 1 year and a third composition for young children above 1 year up to 2 years of age. The set of nutritional compositions may further comprise a fourth composition for young children above 2 years.

Each of the compositions forming part of the set of nutritional compositions comprises probiotics. In the set of nutritional compositions, at least one composition differs from at least another composition in an amount and/or nature of the probiotics present in that composition.

It is understood that the invention also relates to the set of nutritional compositions wherein the first composition for infants between 0 up to 6 months of age, comprises at least 2 (sub) compositions. The at least 2 (sub) compositions are each specifically tailored for children between 0 and up to 1 month of age, above 1 month up to 2 months of age and/or from 3 months and up to 6 months of age. Each or any of the (sub) compositions can have an evolving/varying probiotic content as described in regard to the individual compositions.

Accordingly, in one embodiment, the invention comprises a set of 4, 5, or 6 (sub) compositions, for example targeted at children between birth and less than 1 month, between 1 month and less than 2 or 3 months, between 3 months and less than 6 or 7 months, between 7 and less than 1 year of age, between one year and less than 2 years of age, between 2 years and 3 years or more. Other (sub) compositions with segmentations of age may be considered in the scope of the invention, in particular those segmentations that correspond to the actual evolution of the composition of breast milk as for particular ingredients.

Examples of suitable probiotic micro-organisms used include yeasts such as *Saccharomyces, Debaromyces, Candida, Pichia* and *Torulopsis,* moulds such as *Aspergillus, Rhizopus, Mucor,* and *Penicillium* and *Torulopsis* and bacteria such as the genera *Bifidobacterium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc*, *Weissella, Aerococcus, Oenococcus* and *Lactobacillus.* Specific examples of suitable probiotic micro-organisms are: *Saccharomyces cereviseae, Bacillus coagulans, Bacillus licheniformis, Bacillus subtilis, Bifidobacterium bifidum, Bifidobacterium breve* such as *B. breve* CNCM I-3865, *Bifidobacterium infantis, Bifidobacterium lactis* such as *B. lactis* CNCM I-3446, *Bifidobacterium longum* such as *B. longum* CNCM I-2618, *B*. *longum* BB536 ATCC BAA-999, *B. longum* BL999, *Enterococcus faecium, Enterococcus faecalis, Lactobacillus acidophilus* such as *L*. *acidophilus* L92 FERM BP.4981, *Lactobacillus alimentarius, Lactobacillus casei subsp. casei, Lactobacillus casei Shirota, Lactobacillus curvatus, Lactobacillus delbruckii subsp. lactis, Lactobacillus farciminus, Lactobacillus fermentum* such as *L. fermentum* VRI-033 PCC strain NM 02/31074, *Lactobacillus gasseri, Lactobacillus helveticus* such as *L*. *helveticus* CNCM I-4095, *Lactobacillus johnsonii* such as *L*. *johnsonii* La1 CNCM I-1225, *Lactobacillus paracasei* such as *L. paracasei* ST11 CNCM I-2116, *L. paracasei* 33, *Lactobacillus reuteri* such as *L*. *reuteri* DSM17938, *Lactobacillus rhamnosus* (*Lactobacillus GG*) such as *L*. *rhamnosus* CGMCC 1.3724 ATCC 53103, *Lactobacillus sake, Lactococcus lactis* such as *L*. *lactis* SL31 CNCM I-4154, *Micrococcus varians, Pediococcus acidilactici, Pediococcus pentosaceus, Pediococcus acidilactici, Pediococcus halophilus, Streptococcus faecalis, Streptococcus thermophilus* such as *S*. *thermophilus* CNCM I-3915, *Streptococcus salivarius* such as *S. salivarius* K12 ATCC BAA-1024, *Staphylococcus carnosus,* and *Staphylococcus xylosus,* or any mixtures thereof.

Preferred probiotic bacterial strains include *Lactobacillus rhamnosus* ATCC 53103 obtainable from Valio Oy of Finland under the trade mark LGG, *Lactobacillus rhamnosus* CGMCC 1.3724, *Lactobacillus paracasei* CNCM I-2116, *Lactobacillus reuteri* ATCC 55730 and *Lactobacillus reuteri* DSM 17938 obtainable from BioGaia AB, *Bifidobacterium lactis* CNCM I-3446 sold *inter alia* by the Christian Hansen company of Denmark under the trade mark Bb 12 and *Bifidobacterium longum* ATCC BAA-999 sold by Morinaga Milk Industry Co. Ltd. of Japan under the trade mark BB536, *Bifidobacterium breve* CNCM I-3865, *Bifidobacterium longum* CNCM I-2618, *Lactobacillus acidophilus* L92 FERM BP.4981 from Calpis, *Lactobacillus helveticus* CNCM I-4095, *Lactobacillus johnsonii* La1 CNCM I-1225, *Lactococcus lactis* SL31 CNCM I-4154, *Streptococcus thermophilus* CNCM I-3915, *Streptococcus salivarius* such as *S. salivarius* K12 ATCC BAA-1024 from BLIS, *Lactobacillus paracasei* 33 from Uni President, *L. fermentum* VRI-033 PCC strain NM 02/31074 from Probiomix.

In a preferred embodiment, at least one composition comprises *Bifidobacterium lactis* CNCM I-3446, another composition comprises *Lactobacillus reuteri* DSM 17938.

The probiotics can be fully replicating (live), partially or totally non-replicating (dormant or dead probiotics). The term probiotic in regard to the present invention also comprises parts or fragments of probiotics or inactivated entities or inactivated fragments of probiotics.

The amount of probiotics is usually referred to as cfu (colony forming unit) per g of composition. In case of liquid compositions the equivalent values are considered per ml of composition.

The probiotics are preferably present in an amount of 10³ to 10¹² cfu/g, more preferably 10⁶ to 10¹¹ cfu/g, even more preferably 10⁴ to 10⁹ cfu/g, most preferably 10⁷ to 10⁹ cfu/g composition or per mL of composition.

The amount of probiotics may differ from one composition to another composition of the set of nutritional compositions. For instance, the amount of probiotics may increase with the increasing age range. Thus, the first composition may comprise 10⁴-10⁷ cfu/g of probiotics, the second composition may comprise 10⁶-10⁸ cfu/g of probiotics, the third composition may comprise 10⁷-10⁹ cfu/g of probiotics and the fourth composition may comprise 10⁸-10¹⁰ cfu/g of probiotics.

In one embodiment of the invention the amount of probiotic in the first composition or any of the sub-composition (targeted for example at infants between birth up to 6 months of age or at infants between birth and 1 months, 2 months or 3 months), is disproportionally low compared to the amount in the later compositions (for older infants). In such first composition or sub-compositions, the amount of probiotics can be from 10³ or 10⁴ to 10⁵ or 10⁶ cfu/g. In one embodiment the amount of probiotic in the third and/or fourth composition is from 10⁷ to 10⁹ cfu/g.

In this way, an increasing need of probiotics with increasing age of the infants and young children is addressed.

In a preferred embodiment, at least one, two or three or any of the nutritional compositions of the set comprise 2x10⁷ cfu/g.

Also, the nature of the probiotics may vary from one composition to the other. For instance, one of the composition may comprise *Bifidobacterium lactis* CNCM I-3446, another may comprise *Lactobacillus reuteri* DSM 17938, still another composition may comprise *Streptococcus salivarius* K12 ATCC BAA-1024, and another may comprise *Lactobacillus paracasei* CNCM 1-2116.

In a preferred embodiment of the invention, the probiotics in said first composition are selected from the list comprising *L. helveticus* CNCM I-4095, *B. longum* CNCM I-2618 (NCC2705), *B. breve* NCC2950, *L. reuteri* DSM17938, *L*. *paracasei* ST11 CNCM I-2116 (NCC 6428), *B. longum* BB536 ATCC BAA-999, *B. Longum* BL999 (NCC3001), *L. acidophilus* L92 FERM BP.4981 or any mixtures thereof, preferably in an amount of 10⁴-10⁷ cfu/g, most preferably 10⁶cfu/g.

In another preferred embodiment, the probiotics in said second composition are selected from the list comprising *B. longum* CNCM I-2618 (NCC2705), *B. breve* CNCM I-3865, *L*. *reuteri* DSM17938, *B. lactis* CNCM I-3446, *B. longum* ATCC BAA-999 (Bb536), *L. lactis* SL31 CNCM I-4154 (NCC2287), *L*. *paracasei* ST11 CNCM I-2116 (NCC 6428) or any mixtures thereof, preferably in an amount of 10⁶-10⁸ cfu/g, most preferably 10⁷cfu/g.

In another preferred embodiment, the probiotics in said third and/or fourth composition are selected from the list comprising *Streptococcus salivarius* K12 ATCC BAA-1024, *L*. *rhamnosus* CGMCC 1.3724 ATCC 53103, *Streptococcus thermophilus* CNCM I-3915, *L. paracasei* CNCM I-2116, *L*. *johnsonii* La1 CNCM I-1225 or any mixtures thereof, preferably in an amount of 10⁷-10¹⁰ cfu/g, most preferably 10⁸ cfu/g.

Preferably, the nature of the probiotics is selected for providing complementary benefits. Also, the nature of the probiotics may be selected for addressing issues particular to the age group for which the compositions are intended. For instance, the probiotics used in the first composition may be beneficial for reducing colics, crying time, abdominal pain or for inducing a prevention of allergy. The probiotics used in the second composition may additionally be beneficial for improving immune status, management of allergy, the reduction of allergy manifestation or symptoms and/or the prevention of diarrhea. In one embodiment the probiotics of the 3^{rd} and/or 4^{th} composition are targeted at reducing the risk of obesity later in life (for example by normalizing the growth curve) or prevention of *otitis media* or common cold.

In a preferred embodiment, the probiotics present in the first composition are probiotics which are naturally present in the guts of breast-fed infants.

In a further embodiment, the probiotics present in the third and fourth compositions facilitate weaning. In particular, this facilitation can include the use of specific probiotics that ease the transition to solid food and modify accordingly the intestinal flora.

In one embodiment the probiotic of 2 or 3 of the claimed compositions are the same, possibly varying quantitatively by the amount of probiotic in each composition.

In this way, an array of age-specific nutritional compositions is provided. This overcomes the disadvantages of the known market formulae which generally provide sets of nutritional compositions which comprise the same probiotic load.

It has been found that the set of the invention provides long-term benefits over a period of at least two years, by adapting to the changing needs of the infant/young child during this period of growth.

It has further been found that the set of nutritional compositions work in synergy such that optimal health effects are observed when the nutritional compositions are used consequently. Therefore, using the nutritional compositions independently (i.e. not as part of the set of nutritional compositions) would not achieve the beneficial effects to the same extent.

The beneficial effects range from treatment or prevention of diarrhea, treatment or prevention of gut discomfort, weaning facilitation, maturation of the immune system, prevention or management of allergy, reducing cardiovascular diseases later in life, reducing risk of obesity, reducing risk of infections, ensuring a normal growth curve, improving or insuring optimal cognition, improved immune function and immune defenses, prevention of upper respiratory tract infections like *otitis media* or common cold.

Any of the first, second, third or fourth composition may comprise vitamins, minerals, trace elements, essential fatty acids, lactoferrin, prebiotics, proteins such as proteins associated with milk fat globule membrane (MFGM), TGF-β, hydrolysed or non-hydrolysed protein, insulin, carbohydrates such as lactose, maltodextrin, fat and any mixtures thereof.

The fat in the nutritional compositions may be selected from milk and/or vegetable fat. Typical vegetable fats include palm olein, high oleic sunflower oil, high oleic safflower oil, canola oil, fish oil, coconut oil, bovine milk fat or any mixtures thereof.

In a preferred embodiment, any of the nutritional compositions which form part of the set may further comprise long-chain polyunsaturated fatty acids (LC-PUFA's). LC-PUFA's have been linked to benefits in development of infants and young children. Preferably, the LC-PUFA's are selected from docosahexaenoic acid (DHA), arachidonic acid (ARA), eicosapentaenoic acid (EPA) or any mixtures thereof. Most preferably, the first, second and third nutritional compositions comprise a mixture of docosahexaenoic acid (DHA) and arachidonic acid (ARA). Most preferably, the fourth composition comprises docosahexaenoic acid (DHA).

Any of the compositions of the set of nutritional compositions may also comprise medium chain triglycerides and/or milk fat globule membranes (MFGM).

In the set of nutritional compositions, any of the first, second, third or fourth compositions may further comprise carbohydrates. Suitable carbohydrate sources include lactose, saccharose, maltodextrin, starch and mixtures thereof. In a preferred embodiment, the first and second compositions comprise lactose. Preferably, the amount of lactose in the first and second compositions is between 9.5 and 12 g/100 kcal, preferably between 10 and 11 g/100kcal. The third and fourth compositions preferably comprise a mixture of lactose and maltodextrin. Preferably, the maltodextrin has a DE of 19. Most preferably, the ratio of lactose to maltodextrin in the third and fourth compositions is 70:30.

In the set of nutritional compositions, any of the first, second, third or fourth compositions may further comprise proteins such as intact or hydrolysed protein. Any or all of the compositions of the present invention may comprise hydrolysed protein. Any or all of the composition may also comprise proteins associated with milk fat globule membrane (MFGM), TGF-β, casein, whey, soy protein, or any mixtures thereof. In a preferred embodiment, any or all of the compositions comprise and mixture of whey and casein. Preferably, the ratio of whey to casein in the first composition is 70:30. The second and third compositions preferably have a ratio of whey:casein of 50:50. Preferably, the fourth composition has a whey to casein ratio of 40:60. The protein content in the compositions preferably varies between 1.5 to 2.5 g/100kcal. For instance, the first composition may comprise a protein content of 1.8 to 2.25 g/100kcal. The second composition may comprise a protein content of 1.8 g/100kcal. The third composition may comprise a protein content of 2 g /100kcal. The protein content of the fourth composition is preferably 2.25 g/100kcal.

In the set of nutritional compositions, any of the first, second, third or fourth compositions may further comprise vitamins selected from vitamin A, beta-carotene, vitamin D, vitamin E, vitamin K1, vitamin C, vitamin B1, vitamin B2, niacin, vitamin B6, folic acid, pantothenic acid, vitamin B12, biotin, choline, inositol, taurine, carnitine, or any mixtures thereof.

Additionally, any of the first, second, third or fourth compositions may further comprise minerals selected from sodium, potassium, chloride, calcium, phosphorus, magnesium, manganese or any mixtures thereof.

Trace elements such as iron, iodine, copper, zinc, selenium, fluorine, chromium, molybdenum or any mixtures thereof may also be present in any of the first, second, third or fourth compositions.

In a preferred embodiment, any of the compositions forming part of the set of nutritional composition may comprise lactoferrin.

Additionally, any of the compositions may comprise prebiotics. The prebiotics are preferably present in the compositions in an amount 1 to 20wt%, preferably 2 to 15wt% on a dry matter basis.

A prebiotic is a non-digestible food ingredient that beneficially affects the host by selectively stimulating the growth and/or activity of one or a limited number of bacteria in the colon, and thus improves host health. Such ingredients are non-digestible in the sense that they are not broken down and absorbed in the stomach or small intestine and thus pass intact to the colon where they are selectively fermented by the beneficial bacteria. Examples of suitable prebiotics include certain oligosaccharides, such as fructooligosaccharides (FOS), acacia gum and galactooligosaccharides (GOS). A combination of prebiotics may be used such as 90% GOS with 10% short chain fructooligosaccharides such as the product sold under the trade mark Raftilose® or 10% inulin such as the product sold under the trade mark Raftiline®.

A particularly preferred prebiotic is a mixture of galacto-oligosaccharide(s), N-acetylated oligosaccharide(s) and sialylated oligosaccharide(s) in which the N- acetylated oligosaccharide(s) comprise 0.5 to 4.0% of the oligosaccharide mixture, the galacto-oligosaccharide(s) comprise 92.0 to 98.5% of the oligosaccharide mixture and the sialylated oligosaccharide(s) comprise 1.0 to 4.0% of the oligosaccharide mixture. This mixture is hereinafter referred to as "CMOS-GOS".

Preferably, any of the compositions of the set of nutritional compositions comprise from 2.5 to 15.0 wt% CMOS-GOS on a dry matter basis with the proviso that the composition comprises at least 0.02 wt% of an N-acetylated oligosaccharide, at least 2.0 wt% of a galacto-oligosaccharide and at least 0.04 wt% of a sialylated oligosaccharide.

Suitable N-acetylated oligosaccharides include GalNAcαl, 3Galβl, 4Glc and Galβl,6GalNAcαl,3Galβl,4Glc. The N-acetylated oligosaccharides may be prepared by the action of glucosaminidase and/or galactosaminidase on N-acetyl-glucose and/or N-acetyl galactose. Bqually, N-acetyl-galactosyl transferases and/or N-acetyl-glycosyl transferases may be used for this purpose. The N-acetylated oligosaccharides may also be produced by fermentation technology using respective enzymes (recombinant or natural) and/or microbial fermentation. In the latter case the microbes may either express their natural enzymes and substrates or may be engineered to produce respective substrates and enzymes. Single microbial cultures or mixed cultures may be used. N-acetylated oligosaccharide formation can be initiated by acceptor substrates starting from any degree of polymerisation (DP) from DP=1 onwards. Another option is the chemical conversion of keto-hexoses (e.g. fructose) either free or bound to an oligosaccharide (e.g. lactulose) into N-acetylhexosamine or an N-acetylhexosamine containing oligosaccharide as described in Wrodnigg, T. M.; Stutz, A.E. (1999) Angew. Chem. Int. Ed. 38:827-828.

Suitable galacto-oligosaccharides include Galβl,6Gal, Galβl,6Galβl,4Glc, Galβl,6Galβl,6Glc, Galβl,3Galβl,3Glc, Galβl,3Galβl,4Glc, Galβ1,6Galβ1,6Galβ1,4Glc, Galβ1,6Galβ1,3Galβ1,4Glc, Galβ1,3Galβ1,6Galβ1, 4Glc, Galβl, Galβl,3Galβl,4Glc, Galβl,4Galβl,4Glc, and Galβl,4Galβl,4Glβl,4Glc.

Synthesised galacto-oligosaccharides such as Galβl,6Galβl, 4Glc, Galβl,6Galβl,6Glc, Galβl,3Galβl,4Glc, Galβl,6Galβl,6Galβl,4Glc, Galβl,6Galβl,3Galβl,4Glc and Galβl,3Galβl,6Galβl,4Glc, Galβl,4Galβl,4Glc and Galβl,4Galβl,4Galβl,4Glc and mixtures thereof are commercially available under the trademarks Vivinal® and Elix'or®. Other suppliers of oligosaccharides are Dextra Laboratories, Sigma-Aldrich Chemie GmbH and Kyowa Hakko Kogyo Co.,Ltd. Alternatively, specific glycosyltransferases, such as galactosyltransferases may be used to produce neutral oligosaccharides.

Suitable sialylaled oligosaccharides include NeuAcα2,3Galβl,4Glc and NeuAcα2,6Galβl,4Glc. These sialylated oligosaccharides may be isolated by chromatographic or filtration technology from a natural source such as animal milks. Alternatively the sialylated oligosaccharides may be produced by biotechnology using specific sialyltransferases either by enzyme based fermentation technology (recombinant or natural enzymes) or by microbial fermentation technology. In the latter case microbes may either express their natural enzymes and substrates or may be engineered to produce respective substrates and enzymes. Single microbial cultures or mixed cultures may be used. Sialyl-oligosaccharide formation can be initiated by acceptor substrates starting from any degree of polymerisation (DP) from DP=1 onwards.

The compositions may optionally contain other substances which may have a beneficial effect such as nucleotides, nucleosides. Nucleotides may be selected from cytidine monophosphate (CMP) , uridine monophosphate (UMP), adenosine monophosphate (AMP), guanosine monophosphate (GMP) or any mixtures thereof.

In a preferred embodiment of the invention, the second and third compositions of the set of nutritional compositions are identical.

In an embodiment of the invention, the nutritional compositions are packed in single dose units. Each single dose unit comprises a sufficient amount of nutritional composition to prepare a single serving upon reconstitution with water.

The single serving generally comprises 8 to 35 g, preferably 10 to 30g, most preferably 11 to 28 g of powder to be reconstituted with 80 to 300 mL, preferably between 100mL and 250 mL of water. Alternatively, if the nutritional composition is a concentrate, a single serving includes 1 to 50 mL of concentrate to be diluted with 50 to 250 mL of water.

The invention also pertains to an age-tailored kit for infants and young children. The kit comprises the set of nutritional compositions. The nutritional compositions are packed in the single dose units. Typically, the units comprise 10 to 30 g of powdered nutritional composition or 1 to 50 mL of a concentrate of nutritional composition. Preferably the single dose units are in the form of capsules. The single dose units may also be in the form of stick packs (blister packs)or sachets.

The capsules may be disposable capsules equipped with opening means contained within the capsule to permit draining of the reconstituted formula directly from the capsule into a receiving vessel. The receiving vessel can be for example a feeding bottle for the infants and young children. A method of using capsules for dispensing an infant or young child nutritional composition is described in WO2006/077259. The different nutritional compositions forming part of the set of the invention may be packed into individual capsules and presented to the consumer in multipacks containing a sufficient number of capsules to meet the requirements of an infant for one week for example. Suitable capsule constructions are disclosed in WO2003/059778.

A further aspect of the invention relates to a method for a manufacture of nutritional compositions for infants and young children.

A first step of the method is preparing at least a first, second, third and/or fourth nutritional compositions for infants between 0 up to 6 months, for infants above 6 months up to 1 year, for young children above 1 year up to 2 years and/or for young children above 2 years respectively.

The nutritional compositions may be manufactured in any suitable manner. For example, the nutritional compositions may be prepared by blending together a protein source, a carbohydrate source, and a fat source in appropriate proportions. If used, emulsifiers may be included in the blend. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture.

The liquid mixture may then be thermally treated to reduce bacterial loads. For example, the liquid mixture may be rapidly heated to a temperature in the range of about 80°C to about 110°C for about 5 seconds to about 5 minutes. This may be carried out by steam injection or by heat exchanger; for example a plate heat exchanger. The liquid mixture may then be cooled to about 60°C to about 85°C for example by flash cooling. The liquid mixture may then be homogenised for example in two stages at about 7 MPa to about 40 MPa in the first stage and about 2 MPa to about 14 MPa in the second stage. The homogenised mixture may then be further cooled to add any heat sensitive components such as vitamins and minerals. The pH and solids content of the homogenised mixture are conveniently standardised at this point. The homogenised mixture is transferred to a suitable drying apparatus such as a spray drier or freeze drier and converted to powder. The powder should have a moisture content of less than about 3% by weight. Alternatively, the homogenized mixture is concentrated.

A second step in the method of the present invention consists in selecting for at least the first and the second nutritional compositions at least one probiotic according to age and/or according to a desired effect.

In a preferred embodiment, the second step in the method of the present invention consists in selecting for at least each nutritional compositions or (sub) compositions at least one probiotic according to age and/or according to a desired effect.

The probiotics may be any one of those described herein. The probiotics are selected for instance for desired health benefits. The particular health benefits desired may vary depending on the particular age range. In addition, the amount of probiotics selected may vary according to age or according to the desired effect.

The probiotics may be cultured according to any suitable method and prepared for addition to the nutritional compositions by freeze-drying or spray-drying for example. Alternatively, bacterial preparations can be bought from specialist suppliers such as Christian Hansen and Morinaga already prepared in a suitable form for addition to food products such as nutritional compositions.

In a further step of the present method, the selected probiotics are incorporated to the corresponding nutritional compositions. The probiotics are incorporated such that at least one composition differs from at least one other composition in the amount and/or nature of probiotics present therein.

The probiotics may be added to the powdered nutritional compositions by dry mixing. Alternatively, the probiotics may be added at an earlier stage during preparation of the nutritional compositions. A skilled person is able to determine when the incorporation of the probiotics to the nutritional compositions should occur.

Preferably, the probiotic is incorporated in an amount which varies from 10³ to 10¹² cfu/g or per mL of composition depending on the age and/or the desired effect.

The desired effect may be any of those described herein. These include treatment or prevention of diarrhea, treatment or prevention of gut discomfort, weaning facilitation, maturation of the immune system, prevention or management of allergy, reducing cardiovascular diseases later in life, reducing risk of obesity, reducing risk of infections, ensuring a normal growth curve, improving or insuring optimal cognition, improved immune function and immune defenses, prevention of upper respiratory tract infections like *otitis media* or common cold.

Another facet of the invention therefore relates to an infants and young children nutrition regimen. The infants and young children nutrition regimen comprises feeding an infant between 0 up to 6 months a nutritional composition comprising probiotics, such that the total daily intake is at least 400 kcal, preferably at least 435 kcal.

The infants and young children nutrition regimen the comprises feeding said infant above 6 months up to 1 year a nutritional composition comprising probiotics such that the total daily intake is at least 550 kcal, preferably at least 580 kcal.

The infants and young children nutrition regimen then comprises feeding said infant above 1 year up to 2 years a nutritional composition comprising probiotics such that the total daily intake is at least 750 kcal, preferably at least 765 kcal.

Optionally the infants and young children nutrition regimen comprises feeding said young child over 2 years a nutritional composition comprising probiotics, such that the total daily intake is at least 900 kcal, preferably at least 1000 kcal.

In addition to the nutritional compositions, infants and young children nutrition regimen may include feeding the infants and young children with complementary foods. The complementary foods may be any of the foods commercially available for the corresponding age range. These complementary foods can be for example pureed vegetables, meats, fish, fruits.

In a preferred embodiment, the nutritional compositions fed to the infants and young children encompass more than half of the meals of the infants and young children.

It has been found that infants and young children nutrition regimen provides infants and young children with health benefits at least for the first two years of life.

Thus, by adapting to the changing needs of infants and young children, the present invention provides numerous benefits which have not been achieved by the sets of nutritional compositions known in the art.

The present invention is further illustrated herein by means of the following non-limiting examples.

### Examples

An age-tailored set of nutritional compositions are given in the table below:

| | **Age range** | **0 up to 6 months** | **Above 6th up to 12 months** | **Above 1 up to 2 yr** | **Above 2yr** |
|---|---|---|---|---|---|
| **Basics** | Reconstitution RTD Volume (ml) | 100 to 200 | 230 | 230 | 230 |
| | Energy density (kcal/100 ml) | 63-67 | 63 | 63 | 63 |
| | Content (g/100kcal) | 1.8 - 2.25 | 1.8 | 2 | 2.25 |
| **Protein** | Content (g/l) | 11.3 -15.1 | 11.3 | | |
| | Whey:Casein | 70:30 | 50:50 | 50:50 | 40:60 |
| **Carbohydrates** | Type | Lactose | Lactose | Lactose / MD DE19 (70:30) | Lactose / MD DE19 (70:30) |
| | Content (g/100kcal) | 9.7 to 11.6 | 10.6 | 10.6 | 14.2 |
| | Content (g/l) | 65.0 to 73.5 | 66.8 | | |
| **Lipids** | Type | Milk & Veg. | Milk & Veg. | Milk & Veg. | Fat mix* |
| | Content (g/100kcal) | 5.1 to 5.8 | 5.6 | 5.6 | 4 |
| | content (as % of total energy) | 45.9 to 52.2 | 45.8 | 50.4 | 50.4 |
| | Content (g/l) | 32.1 to 38.9 | 35.3 | | |
| | LC-PUFA | DHA + ARA | DHA + ARA | DHA + ARA | DHA 0.3%tfa ** |
| **Probiotics** | Type | *B.lactis CNCM I-3446* and/or *B*. *longum CNCM I-2618* | *L. reuteri DSM-17938* | *L*. *paracase i CNCM I-2116* | *L*. *rhamnos* us ATCC 53103 and/or S. *salivar ius* K12 DSM1468 6 |
| | Content | 2x10⁵cfu/g | 2x10⁷cfu/ g | 2x10⁸cfu /g | 2x 10⁸cfu/ g |
| **Prebiotics** | | | | | Optiona lly FOS and/or acacia gum |
| **Nucleotide** | CMP (mg/100 kcal) | 1.1 | - | | - |
| | UMP | 0.7 | - | | - |
| | AMP | 0.7 | - | | - |
| | GMP | 0.2 | - | | - |
| **Minerals(/100kcal)** | Na (mg) | 25 to 37.5 | 25 | 25 | - |
| | K (mg) | 80 to 95 | 80 | 80 | - |
| | *Na*/*K (molar ratio)* | 0.53-0.67 | 0.53 | 0.53 | - |
| | *(Na+K)* /*Cl molar ratio* | *1.71- 1.81* | *1.71* | *1.71* | - |
| | Cl (mg) | 65 to 80 | 65 | 65 | - |
| | Ca (mg) | 60 | 60 | 80 | 80 |
| | P (mg) | 33 | 33 | 50 | 50 |
| | Mg (mg) | 7 | 7 | 10 | 10 |
| | Mn (µg) | 5 | 5 | - | - |
| | Ca/P | 1.8 | 1.8 | 1.6 | 1.6 |
| **Vitamins (/100kcal)** | Vit. A (mg RE) | 0.09 to 01125 | 0.09 | 0.06 | 0.06 |
| | Vit. D (mg) | 0.0015 | 0.0015 | 0.0018 | 0.0018 |
| | Vit. E (mg) | 1.3 | 1.3 | 1.3 | 1.3 |
| | Vit. K1 (µg) | 8 | 8 | 4 | 4 |
| | Vit. C (mg) | 15 | 15 | 10 | 10 |
| | Vit. B1 (mg) | 0.07 to 0.1 | 0.1 | 0.08 | 0.08 |
| | Vit. B2 (mg) | 0.1 | 0.1 | 0.08 | 0.08 |
| | Niacin (mg) | 0.5 | 0.5 | 0.8 | 0.8 |
| | Vit. B6 (mg) | 0.05 | 0.05 | 0.07 | 0.07 |
| | Folic acid (µg) | 15 to 16 | 15 | 15 | 15 |
| | Pantothenic Acid (mg) | 0.7 to 0.8 | 0.8 | 0.4 | 0.4 |
| | Vit. B12 (µg) | 0.2 | 0.2 | 0.15 | 0.15 |
| | Biotin (µg) | 2 | 2 | 1.5 | 1.5 |
| | Choline (mg) | 20 | 20 | 30 | 30 |
| | Inositol (mg) | 25 | 20 | - | - |
| | Taurine (mg) | 8 | 6 | - | - |
| | Carnitine (mg) | 1.5 | - | - | - |
| **Trace Elements (/100kcal)** | Fe (mg) | 0.7 | 1 | 1 | 1 |
| | I (µg) | 15 to 20 | 20 | 15 | 15 |
| | Cu (mg) | 0.06 to 0.08 | 0.06 | 0.05 | 0.05 |
| | Zn (mg) | 1 to 1.2 | 0.8 | 0.6 | 0.6 |
| | Se (µg) | 3 to 4 | 3 | 3.5 | 3.5 |

| | | | | | |
|---|---|---|---|---|---|
| * fat mix follows AHA: sat. Fat <7%E + polyuns. <10%E LA/ALA 5.0 ** tfa | | | | | |

Having thus described the present invention in detail and the advantages thereof, it is to be understood that the detailed description is not intended to limit the scope of the invention thereof.

What is desired to be protected by letters patent is set forth in the following claims.

## Claims

1. A set of nutritional compositions for infants and young children comprising:
- a first composition for infants between 0 up to 6 months,
- a second composition for infants above 6 months up to 1 year,
- a third composition for young children above 1 year up to 2 years; and wherein at least the first and second compositions comprise probiotics and wherein said first and second compositions differ from each other in the amount and/or nature of probiotics present therein.

2. The set of nutritional compositions for infants and young children according to claim 1 further comprising a fourth composition for young children above 2 years.

3. The set of nutritional compositions for infants and young children according to claims 1 or 2, wherein the third composition and/or the fourth composition comprise probiotics.

4. The set of nutritional compositions for infants and young children according to any of the above claims, wherein the amount and/or nature of probiotics present in each composition are selected according to an age and/or according to a desired effect of the infants and young children.

5. The set of nutritional compositions for infants and young children according to any of the above claims, wherein the first composition for infants between 0 up to 6 months comprises at least two sub-compositions each specifically tailored for infants between 0 up to 1 month of age and above 1 month up to 2 months of age and/or from 3 months up to 6 months of age.

6. The set of nutritional compositions for infants and young children according to any of the above claims, wherein the probiotics are selected from any of yeasts such as *Saccharomyces, Debaromyces, Candida, Pichia* and *Torulopsis,* moulds such as *Aspergillus, Rhizopus, Mucor,* and *Penicillium* and *Torulopsis* and bacteria such as the genera *Bifidobacterium, Bacteroides, Clostridium, Fusobacterium, Melissococcus, Propionibacterium, Streptococcus, Enterococcus, Lactococcus*, *Staphylococcus, Peptostrepococcus, Bacillus, Pediococcus, Micrococcus, Leuconostoc, Weissella, Aerococcus*, *Oenococcus* and *Lactobacillus,* preferably the probiotics are selected from any of *Saccharomyces cereviseae, Bacillus coagulans, Bacillus licheniformis, Bacillus subtilis, Bifidobacterium bifidum, Bifidobacterium breve* such as *B. breve* CNCM I-3865, *Bifidobacterium infantis, Bifidobacterium lactis* such as *B. lactis* CNCM I-3446, *Bifidobacterium longum* such as *B. longum* CNCM I-2618, *B. longum* BB536 ATCC BAA-999, *B. longum* BL999, *Enterococcus faecium, Enterococcus faecalis, Lactobacillus acidophilus* such as *L. acidophilus* L92 FERM BP.4981, *Lactobacillus alimentarius, Lactobacillus casei subsp. casei, Lactobacillus casei Shirota*, *Lactobacillus curvatus*, *Lactobacillus delbruckii subsp. lactis, Lactobacillus farciminus, Lactobacillus fermentum* such as *L. fermentum* VRI-033 PCC strain NM 02/31074, *Lactobacillus gasseri, Lactobacillus helveticus* such as *L. helveticus* CNCM I-4095, *Lactobacillus johnsonii* such as *L. johnsonii* La1 CNCM I-1225, *Lactobacillus paracasei* such as *L. paracasei* ST11 CNCM I-2116, *L*. *paracasei* 33, *Lactobacillus reuteri* such as *L. reuteri* DSM17938, *Lactobacillus rhamnosus* (*Lactobacillus GG*) such as *L. rhamnosus* CGMCC 1.3724 ATCC 53103, *Lactobacillus sake, Lactococcus lactis* such as *L*. *lactis* SL31 CNCM I-4154, *Micrococcus varians, Pediococcus acidilactici, Pediococcus pentosaceus, Pediococcus acidilactici, Pediococcus halophilus, Streptococcus faecalis, Streptococcus thermophilus* such as *S. thermophilus* CNCM I-3915, *Streptococcus salivarius* such as *S*. *salivarius* K12 ATCC BAA-1024, *Staphylococcus carnosus*, and *Staphylococcus xylosus,* or any mixtures thereof.

7. The set of nutritional compositions for infants and young children according to any of the above claims, wherein the probiotics are present in the compositions in an amount ranging from 10³ to 10¹² cfu/g, more preferably 10⁶ to 10¹¹ cfu/g, even more preferably 10⁴ to 10⁹ cfu/g, most preferably 10⁷ to 10⁹ cfu/g composition or per mL of composition.

8. The set of nutritional compositions for infants and young children according to any of the above claims, wherein the probiotics present in the first composition are probiotics which are naturally present in the guts of breast-fed infants.

9. The set of nutritional compositions for infants and young children according to any of 2 to 8, wherein the probiotics present in the third and fourth compositions facilitate weaning.

10. The set of nutritional compositions for infants and young children according to any of 2 to 9, wherein any of the first, second, third or fourth composition comprises vitamins, minerals, trace elements, essential fatty acids, lactoferrin, prebiotics, proteins such as proteins associated with milk fat globule membrane (MFGM), TGF-β, hydrolysed or non-hydrolysed protein, insulin, carbohydrates such as lactose, maltodextrin, fat and any mixtures thereof.

11. The set of nutritional compositions for infants and young children according to any of the above claims, wherein the probiotics in the first composition are selected from the list comprising *L. helveticus* CNCM I-4095, *B. longum* CNCM I-2618, *B. breve* NCC2950, *L*. *reuteri* DSM17938, *L. paracasei* ST11 CNCM I-2116, *B*. *longum* BB536 ATCC BAA-999, *B. Longum* BL999, *L*. *acidophilus* L92 FERM BP.4981 or any mixtures thereof, preferably in an amount of 10⁴-10⁷ cfu/g, most preferably 10⁶cfu/g.

12. The set of nutritional compositions for infants and young children according to any of the above claims, wherein the probiotics in the second composition are selected from the list comprising *B. longum* CNCM I-2618, *B. breve* CNCM I-3865, *L. reuteri* DSM17938, *B*. *lactis* CNCM I-3446, *B. longum* ATCC BAA-999 (Bb536), *L*. *lactis* SL31 CNCM I-4154, *L. paracasei* ST11 CNCM I-2116 or any mixtures thereof, preferably in an amount of 10⁶-10⁸ cfu/g, most preferably 10⁷cfu/g.

13. The set of nutritional compositions for infants and young children according to any of the above claims, wherein the probiotics in the third and/or the fourth composition are selected from the list comprising *Streptococcus salivarius* K12 ATCC BAA-1024, *L*. *rhamnosus* CGMCC 1.3724 ATCC 53103, *Streptococcus thermophilus* CNCM I-3915, *L. paracasei* CNCM I-2116, *L*. *johnsonii* La1 CNCM I-1225 or any mixtures thereof, preferably in an amount of 10⁷-10¹⁰ cfu/g, most preferably 10⁸ cfu/g.

14. The set of nutritional compositions for infants and young children according to any of the above claims, wherein the second and third compositions are identical.

15. A use of the set of nutritional compositions for infants and young children according to any of the above claims for providing a health benefit to infants and young children, the health benefit selected from treatment or prevention of diarrhea, treatment or prevention of gut discomfort, weaning facilitation, maturation of the immune system, prevention or management of allergy, reducing cardiovascular diseases later in life, reducing risk of obesity, reducing risk of infections, ensuring a normal growth curve, improving or insuring optimal cognition, improved immune function and immune defenses, prevention of upper respiratory tract infections like *otitis media* or common cold.

16. A nutrition kit for infants and young children comprising a set of nutritional compositions according to any of claims 1 to 14, wherein the nutritional compositions are packed in single dose units, wherein each single dose unit comprising sufficient nutritional composition to prepare a single serving upon reconstitution with water.

17. The nutrition kit for infants and young children according to claim 16, wherein the single dose unit is a capsule.

18. A method for the manufacture of a set of nutritional compositions for infants and young children comprising the steps of:
- preparing at least a first, a second, a third nutritional composition for infants between 0 up to 6 months, for infants above 6 months up to 1 year, for young children above 1 year up to 2 years respectively,
- selecting for at least the first nutritional composition and the second nutritional composition at least one probiotic according to age and/or according to a desired effect,
- incorporating said selected probiotic to the corresponding nutritional composition, such that at least said first composition differs from at least said second composition in an amount and/or nature of probiotics present therein.

19. The method of claim 18, wherein the probiotic is incorporated in an amount which varies from 10³ cfu/g to 10¹² cfu/g or per mL of composition depending on the age and/or the desired effect.

20. The method according to claim 18 or 19, wherein the desired effect is any of treatment or prevention of diarrhea, treatment or prevention of gut discomfort, weaning facilitation, maturation of the immune system, prevention or management of allergy, reducing cardiovascular diseases later in life, reducing risk of obesity, reducing risk of infections, ensuring a normal growth curve, improving or insuring optimal cognition, improved immune function and immune defenses, prevention of upper respiratory tract infections like *otitis media* or common cold.

21. An infants and young children nutrition regimen comprising:
- feeding said infant between 0 up to 6 months a nutritional composition comprising probiotics such that the daily caloric intake is at least 400 kcal, preferably at least 435 kcal,
- feeding said infant above 6 months up to 1 year a nutritional composition comprising probiotics such that the daily caloric intake is at least 550 kcal, preferably at least 580 kcal; and
- feeding said young child above 1 year up to 2 years a nutritional composition comprising probiotics such that the daily caloric intake is at least 750 kcal, preferably at least 765 kcal,
wherein at least one of the compositions differs from at least another one of the compositions in the nature and/or amount of probiotics present therein.

22. The infants and young children nutrition regimen according to claim 21 further comprising:
- feeding said young child over 2 years a nutritional composition comprising probiotics such that the daily caloric intake is at least 900 kcal, preferably at least 1000 kcal.

23. The infants and young children nutrition regimen according to any of claims 21 to 22, wherein the compositions fed to the infants and young children encompass more than half of his meals.

24. The infants and young children nutrition regimen according to an of claims 21 to 23, wherein the variation of the probiotic content in said compositions evolves according to an age of the infants and young children.
